# EUROPEAN PATENT SPECIFICATION

(11) **EP 2 856 953 B1**
(45) Date of publication and mention of the grant of the patent: **07.10.2015**
(21) Application number: 13005806.8
(22) Date of filing: 13.12.2013
(51) Int. Cl.: A61B 17/24, A46B 15/00, A61B 17/32

(54) **Intraoral cleaner**
Intraoraler Reiniger
Aspirateur intraoral

(30) Priority: 07.10.2013 JP 2013209863
(43) Date of publication of application: 08.04.2015
(73) Proprietor: Shikien Co. ltd., Niigata-shi, Niigata 956-0057 (JP)
(72) Inventor: Tanaka, Michio, Niigata-shi; Niigata (JP)
(74) Representative: Angerhausen, Christoph

(56) References cited:
- EP-A2- 2 446 840
- WO-A1-01/34042
- US-A- 2 491 274
- US-A1- 2007 178 273
- US-A1- 2009 131 960

## Description

### BACKGROUND OF THE INVENTION

### Field of the invention

The present invention relates to an intraoral cleaner for cleaning the interior of the oral cavity, as defined in US 2009/131960 A1 which has been used to formulate the preamble part of claim 1.

Conventionally, as a tongue cleaner, there has been disclosed a tongue cleaner (e.g. JP-A-2012-95995) composed of: a flat and thin head portion; d sheets arranged on both sides of the head portion; a rod-shaped grip; and a ring portion integrally formed together with the rod-shaped grip such that the sheets can be fixed on a side surface of the head portion.

Further, there has also been disclosed a 360-degree cylindrical toothbrush (e.g. JP-A-2005-103225) equipped with annular bristle brush members. This toothbrush can be used in a 360-degree range.

### SUMMARY OF THE INVENTION

As for the tongue cleaner disclosed in JP-A-2012-95995, as shown in FIG.19 to FIG.21, a head portion 101 is flat and thin, and has rough-surfaced sheets 104, 105 arranged on both sides thereof. Further, a rod-shaped grip 102 is integrally formed together with a ring portion 106 such that the sheets 104, 105 can be fixed on a side surface of the head portion 101. Here, the ring portion 106 for fixing the sheets 104, 105 on the side surface of the head portion 101, cannot contribute to cleaning. Therefore, when cleaning regions in the mouth other than tongue or teeth (e.g. the upper jaw in the mouth cavity, the rear side of the cheek, gums, and areas between teeth and lip), the side surface of the head portion 101, particularly a front end region of the side surface of the head portion 101 cannot be used to perform cleaning regardless of the fact that the side surface of the head portion 101 can most easily touch a region to be cleaned, and that the front end region of the side surface of the head portion 101 is easy to use. That is, although this tongue cleaner is suitable for cleaning tongue, it is not suitable for cleaning the entire region in the oral cavity other than the tongue.

Further, burrs or the like formed along edges 104A, 105A of the sheets 104, 105 will both be exposed if simply removing the ring portion 106 from the structure. In such case, the burrs or the like may injure the inner side of the mouth.

The 360-degree cylindrical toothbrush disclosed in JP-A-2005-103225 does not include an element corresponding to the ring portion disclosed in JP-A-2012-95995. In fad., this toothbrush can be used in a 360-degree range. However, even this toothbrush is not suitable for wholly cleaning the interior of the oral cavity due to the fact that no bristle brush member is provided on a front end section of a head portion which is most effective for thoroughly cleaning regions in the mouth other than teeth (e.g. the upper jaw in the mouth cavity, the rear side of the cheek, gums, and areas between teeth and lip. Moreover, even the bristle brush members of this cylindrical toothbrush mainly designed to brush teeth, are not suitable for cleaning the interior of the oral cavity where soft mucous membranes exist.

In addition, due to structural complexities and an increased amount of time and effort for manufacturing, it is not realistic to combine the cylindrical tooth brush disclosed in JP-A-2005-103225 with the sheets of the tongue cleaner disclosed in JP-A-2012-95995 that are equipped with the loop-shaped linear members suitable for cleaning soft mucous membranes.

It is an object of the present invention to provide an intraoral cleaner capable of widely cleaning the interior of the oral cavity due to the fact that a head portion thereof does not include an element corresponding to a ring portion found in the conventional tongue cleaners.

Further, it is also an object of the present invention to provide an intraoral cleaner capable of cleaning the interior of the oral cavity without injuring soft mucous membranes therein.

The invention as defined in claim 1 is an intraoral cleaner including: a rod-shaped grip; and a thin and flat head portion provided on a front end of the rod-shaped grip, wherein the head portion has: a pouch-shaped sheet; and a core portion filling an interior of the pouch-shaped sheet, such pouch-shaped sheet having, on an entire outer surface thereof, a plurality of loop-shaped linear members for scraping out dirt inside an oral cavity, in which the pouch-shaped sheet comprising two pieces of sheet material further has a joint section formed by a fused portion of the two pieces of sheet material on an inner side of the pouch-shaped sheet.

Preferably, the pouch-shaped sheet and the core portion are tightly adhered to each other.

Also preferred the pouch-shaped sheet is formed into the shape of a pouch having a portion broader than an opening section.

The intraoral cleaner of the invention provides an intraoral cleaner capable of widely cleaning the interior of the oral cavity. That is, the head portion of this intraoral cleaner is composed of the pouch-shaped sheet and the core portion inside the pouch-shaped sheet, thereby allowing the entire outer surface of the head portion to be covered by the soft loop-shaped linear members. The intraoral cleaner is capable of cleaning the interior of the oral cavity without hurting soft mucous membranes in the oral cavity. This is possible due to the fact that the joint section of the pouch-shaped sheet is now formed inward such that burrs are prevented from being exposed.

The invention preferably also provides a hygienic intraoral cleaner capable of inhibiting bacteria inside the pouch-shaped sheet from proliferating due to the fact that the pouch-shaped sheet and the core portion are tightly adhered to each other.

The invention further may provide an easy-to-use intraoral cleaner. That is, the pouch-shaped sheet is formed narrower than a wide portion on a front end region thereof, thus making it possible to downsize a portion that is directly connected to the head portion but does not contribute to cleaning.

Further, the invention comprises a method for manufacturing the intraoral cleaner according to claim 1, in which the head portion is formed through: a step of forming the pouch-shaped sheet by placing sheet materials on top of each other with rough surfaces thereof facing each other, such rough surfaces being first surfaces of the sheet materials and having a plurality of linear members, and then fusing the sheet materials such that a resultant fused portion conforms to the outer shape of the head portion; a step of turning the pouch-shaped sheet inside out; and a step of forming the core portion inside the pouch-shaped sheet through injection molding.

The method allows an intraoral cleaner to be easily manufactured. That is, the pouch-shaped sheet is at first formed inside out and then turned inside out again, followed by forming the core portion inside the pouch-shaped sheet through injection molding.

### BRIEF DESCRIPTION OF THE DRAWINGS

FIG.1 is a perspective view of an intraoral cleaner of the present invention.
FIG.2 is a cross-sectional view of the intraoral cleaner, taken on line A-A in FIG.1.
FIG.3 is a cross-sectional view of the intraoral cleaner, taken on line B-B in FIG.1.
FIG.4 is an enlarged view of an essential portion of a head portion of the intraocular cleaner.
FIG.5A is a perspective view of a bundle of linear members that have not yet been convexed and concaved.
FIG.5B is a perspective view of the bundle of the linear members that have been convexed and concaved.
FIG.6 is an enlarged partial view of the linear members being convexed and concaved.
FIG.7 is a diagram showing a first step of forming a pouch-shaped sheet of the intraoral cleaner.
FIG.8 is a diagram showing a second step of forming the pouch-shaped sheet of the intraoral cleaner.
FIG.9 is a diagram showing a third step of forming the pouch-shaped sheet of the intraoral cleaner.
FIG.10A is a first motion diagram of a flow chart of a fourth step of forming the pouch-shaped sheet of the intraoral cleaner.
FIG.10B is a second motion diagram of the flow chart of the fourth step of forming the pouch-shaped sheet of the intraoral cleaner.
FIG.10C is a third motion diagram of the flow chart of the fourth step of forming the pouch-shaped sheet of the intraoral cleaner.
FIG.10D is a fourth motion diagram of the flow chart of the fourth step of forming the pouch-shaped sheet of the intraoral cleaner.
FIG.10E is a fifth motion diagram of the flow chart of the fourth step of forming the pouch-shaped sheet of the intraoral cleaner.
FIG.10F is a sixth motion diagram of the flow chart of the fourth step of forming the pouch-shaped sheet of the intraoral cleaner.
FIG.11 is a top view of molds used in the present invention.
FIG.12 is a transverse cross-sectional view of the molds coupled to each other.
FIG.13 is a top view of a core base member with the pouch-shaped sheet put thereon.
FIG.14 is a top view of the core base member without the pouch-shaped sheet.
FIG.15 is a cross-sectional view of the core base member, taken on line A-A in FIG.14.
FIG.16 is a longitudinal cross-sectional view of the core base member with the pouch-shaped sheet put thereon.
FIG.17 is a longitudinal cross-sectional view of a state where the core base member with the pouch-shaped sheet put thereon is received in the molds.
FIG.18 is a longitudinal cross-sectional view of the core base member with the pouch-shaped sheet put thereon; and the molds receiving the same, after performing injection molding.

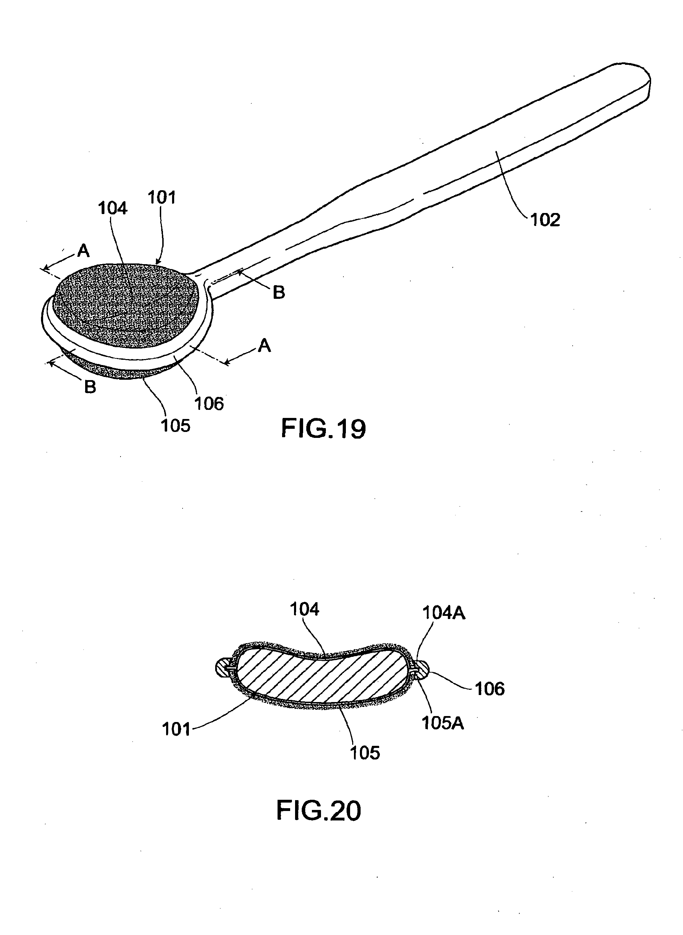

FIG.19 is a perspective view of a conventional tongue cleaner.
FIG.20 is a cross-sectional view of the conventional tongue cleaner, taken on line A-A in FIG.19.

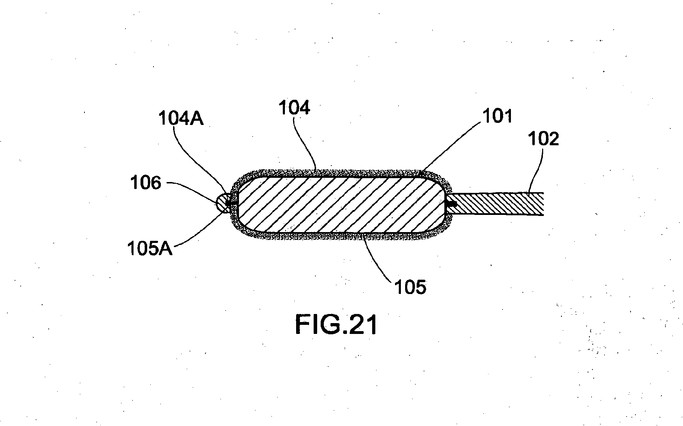

FIG.21 is a cross-sectional view of the conventional tongue cleaner, taken on line B-B in FIG.19.

### DETAILED DESCRIPTION OF THE INVENTION

A preferable embodiment of the present invention is described hereunder with reference to the accompanying drawings. However, this embodiment does not limit the contents of the present invention as set forth in the claims. Further, not all elements described hereunder are necessarily the essential elements of the present invention.

### (First embodiment)

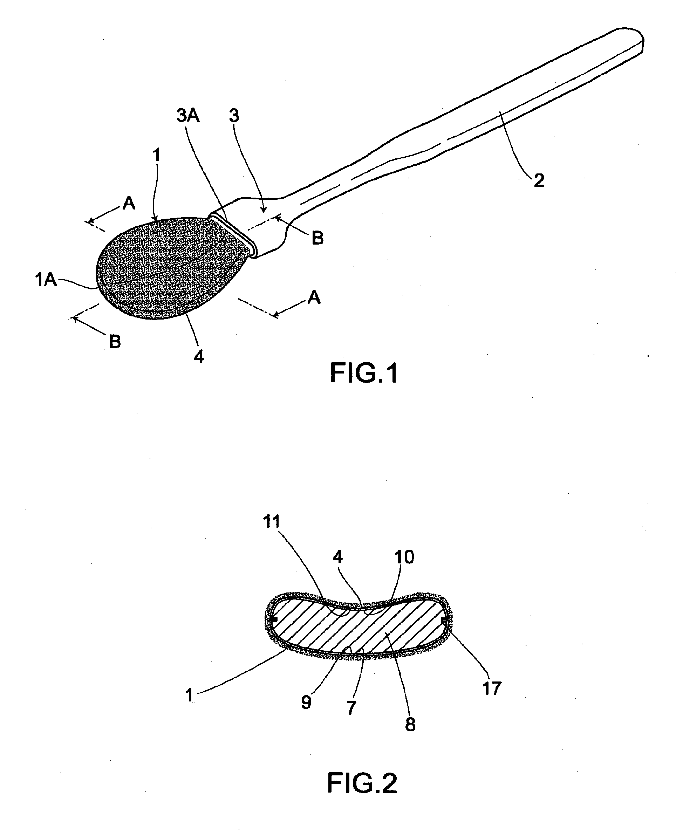

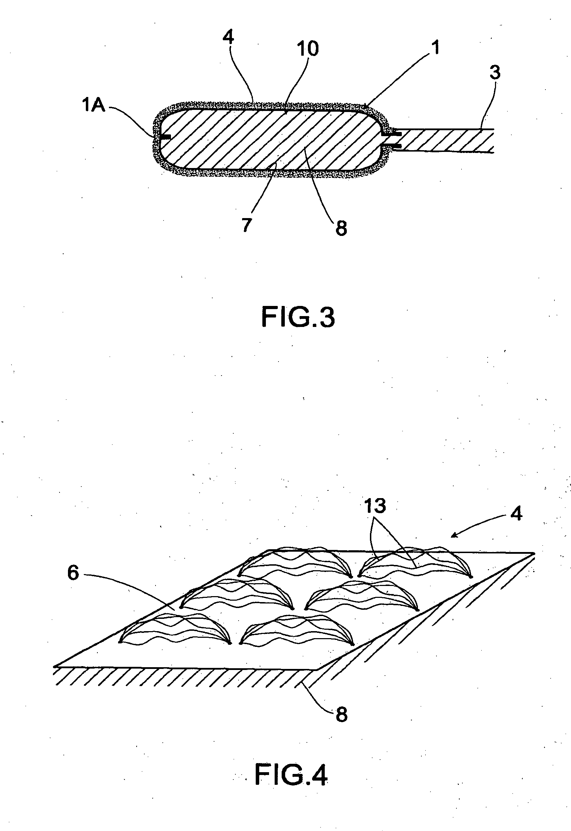

As shown in FIG.1 to FIG.4, an intraoral cleaner of the present invention includes a head portion 1 as a cleaner main body and a rod-shaped grip 2 that are substantially aligned in a straight line. Formed between the head portion 1 and the rod-shaped grip 2 is a head base portion 3 of a shape tapering toward the rod-shaped grip 2 from the end of the head portion 1. This head portion 1 is formed flat and thin. Particularly, one surface of the head portion 1 (i.e. an under surface shown in FIG.1 and FIG.2) is formed into a convex curved surface 9 where a mound portion 7 is provided along the longitudinal direction of the rod-shaped grip 2. Further, the other surface of the head portion 1 (i.e. an upper surface) is formed into a concave curved surface 11 where a concave portion 10 is provided along the longitudinal direction of the rod-shaped grip 2. Moreover, the head portion 1 includes: a pouch-shaped sheet 4 whose entire outer surface is provided with loop-shaped linear members 13; and a core portion 8 filling the interior of the pouch-shaped sheet 4.

As for the aforementioned structure, the entire outer surface of the head portion 1, particularly the side surface of a front end portion 1A of the head portion 1, is covered with soft loop-shaped linear members, thus making it possible to widen the range of purpose of use by: cleaning regions in the mouth other than teeth such as the upper jaw in the mouth cavity, the rear side of the cheek, gums, and areas between teeth and lip; or whitening the teeth.

Further, since the head portion 1 does not include a ring portion, not only dimensions such as the thickness of the head portion 1 itself can be reduced, but the teeth can also be prevented from coming into contact with the ring portion that is harder than the sheet.

In the present embodiment, the linear members 13 are provided in the order of 300 deniers. Further, about 2,000 loops of the linear members 13 are provided per 1 cm square on the outer surface of the pouch-shaped sheet 4.

When viewed from top, the head portion 1 is formed into a gentle "R" shape having a bulging lower area. Here, the width of such shape on the rod-shaped grip 2 side is formed narrower than the largest width thereof on the front end side.

Each linear member 13 is formed into a wavy shape having concavities and convexities whose orientations and shapes are not identical to one another. The linear members 13 are provided on rough surfaces 6 of sheet materials 5 serving as materials of the pouch-shaped sheet 4. And the wavy shapes thereof are formed as follows.

As for each piece of the sheet material 5, formed on the rough surface 6 side thereof are the linear members 13. Particularly, the linear members 13 are grouped together into bundles each composed of a plurality (e.g. in a number of 10 or so) of the corresponding linear members 13. Here, each bundle includes: a pair of raised and protruded portions 13A, 13B; and a bridge portion 13C that is provided between the front ends of the protruded portions 13A, 13B, and whose arc-like near-central section protrudes upward.

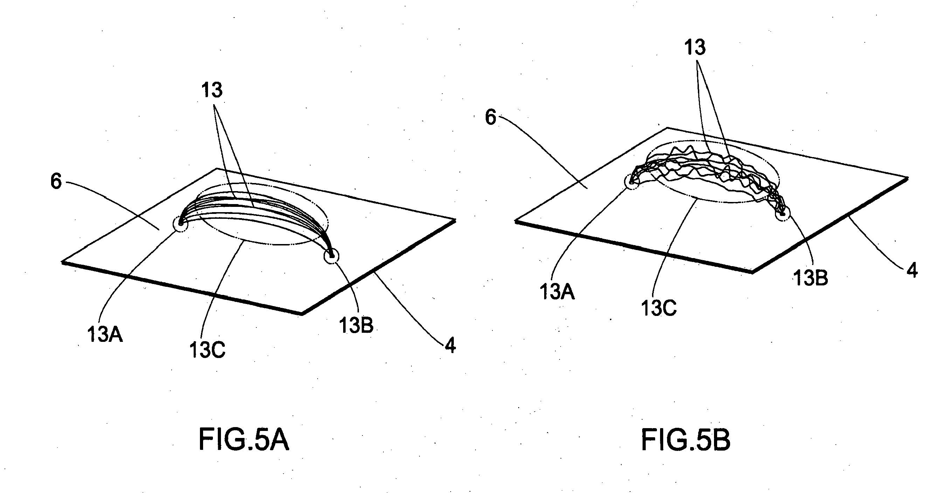

The shapes of the linear members 13 are substantially identical to each other before forming the concavities and convexities whose orientations and shapes are not identical to one another (e.g. FIG.5A).

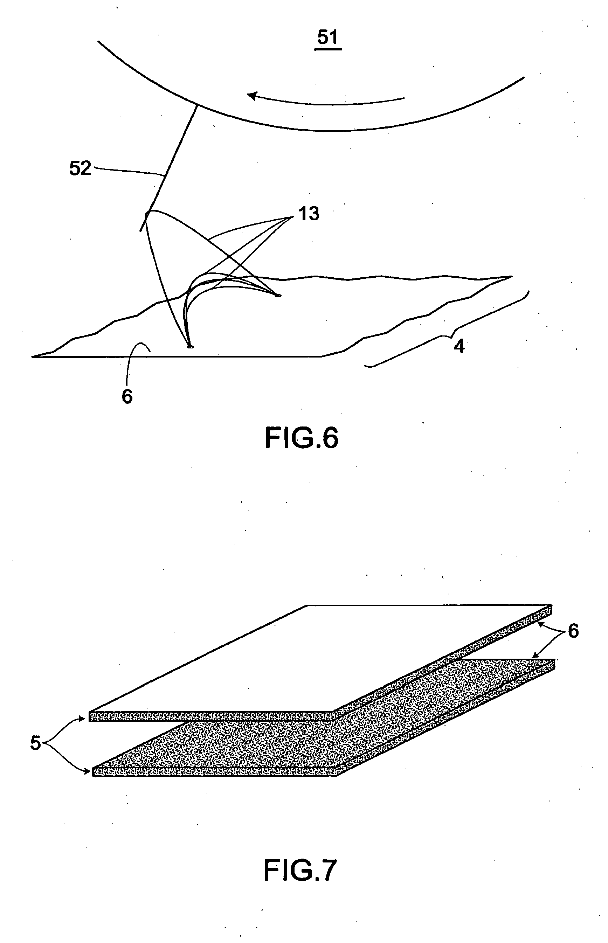

A rotary brush composed of: a cylindrical roller 51; and a plurality of radially protruding thin needles 52 that are randomly arranged on the roller 51, is to be pressed against each loop-shaped linear member 13 and then rotated, thereby allowing each linear member 13 to plastically deform as a result of being individually hooked by the needles 52, thus forming the concavities and convexities whose orientations and shapes are not identical to one another (e.g. FIG.6).

Particularly, it is desired that a plurality of the rotary brushes be individually used to sequentially perform deformation processing, thus increasing the randomness in the nonidentical orientations and shapes of the concavities and convexities.

The head portion 1 is structured as follows. That is, as shown in FIG.2, the head portion 1 includes: the pouch-shaped sheet 4; and the core portion 8 filling the interior of the pouch-shaped sheet 4.

It is desired that a burr formed on a joint section of the pouch-shaped sheet 4 be hidden inward.

As for the head portion 1, a method of forming the pouch-shaped sheet 4 is described hereunder with reference to FIG.7 through FIGs.10.

Two pieces of the sheet material 5 with the loop-shaped linear members 13 provided on the first surfaces thereof (i.e. the rough surfaces 6), are to be placed on top of the other in a manner such that the rough surfaces 6 having the linear members 13 face each other (e.g. FIG.7).

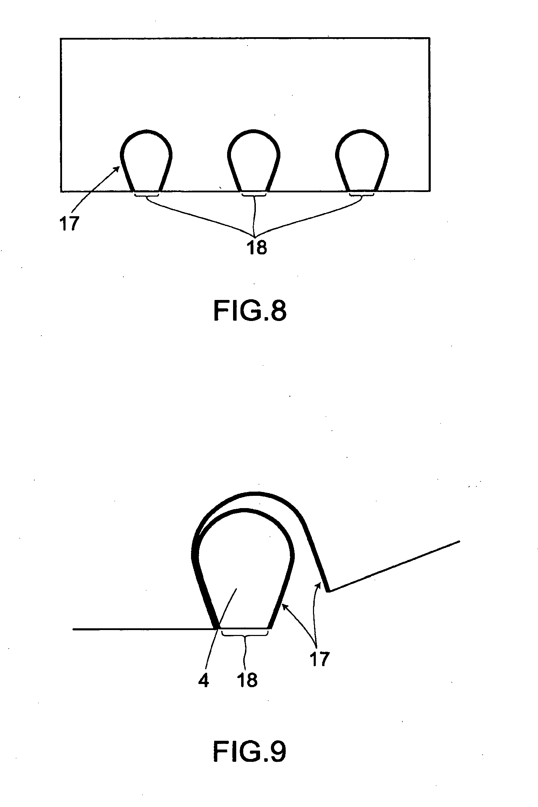

With the two pieces of the sheet material 5 being sandwiched, a melt-adhesion device (not shown) is then used to fuse, with heat, the sheet materials 5 to each other such that one or more resultant fused portions 17 conform to the outer shape of the head portion 1 (e.g. FIG.8). Here, the edge regions of the sheet materials 5 are not fused to each other due to the fact that these regions shall compose an opening section 18 of the pouch.

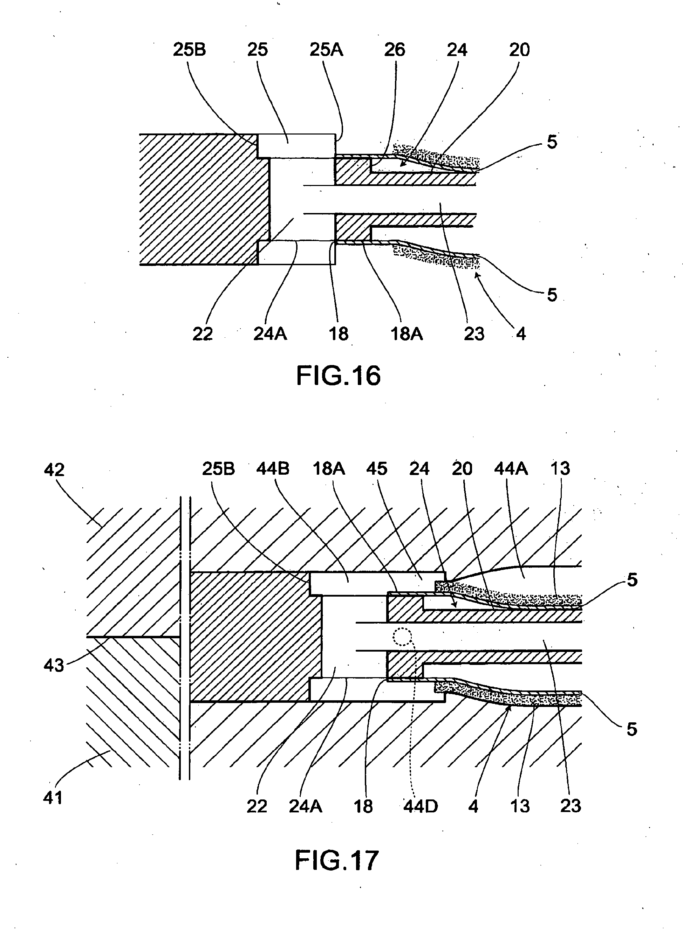

Further, in the present embodiment, no linear member 13 is provided on the entire circumference of a band-shaped region 18A in the opening section 18. As shown in FIG.1, for example, the entire longitudinal area of the outer circumference of the band-shaped region 18A; or at least a portion thereof, is sealed by a sealing portion 3A formed on the front end of the head base portion 3. That is, the opening section 18 is sealed by such sealing portion 3A. In the present embodiment, as shown in FIG.16, for example, also sealed by the sealing portion 3A are: the entire longitudinal area of the band-shaped region 18A; and a section of an area close to the band-shaped region 18A where the linear members 13 are provided. Thus, the opening section 18 of the pouch-shaped sheet 4 is reliably sealed and fixed by the sealing portion 3A such that the opening section 18 shall not open or move.

By performing separation at the resultant fused portion(s) 17, the pouch-shaped sheet 4 now having the resultant fused portion 17 as the joint section is formed (e.g.

FIG.9). As for a method of performing separation at the resultant fused portion(s) 17, the separation may actually be performed either automatically using a punching machine or manually.

The pouch-shaped sheet 4 thus formed is then turned inside out such that the rough surface 6 provided with the linear members 13 now becomes the outer surface.

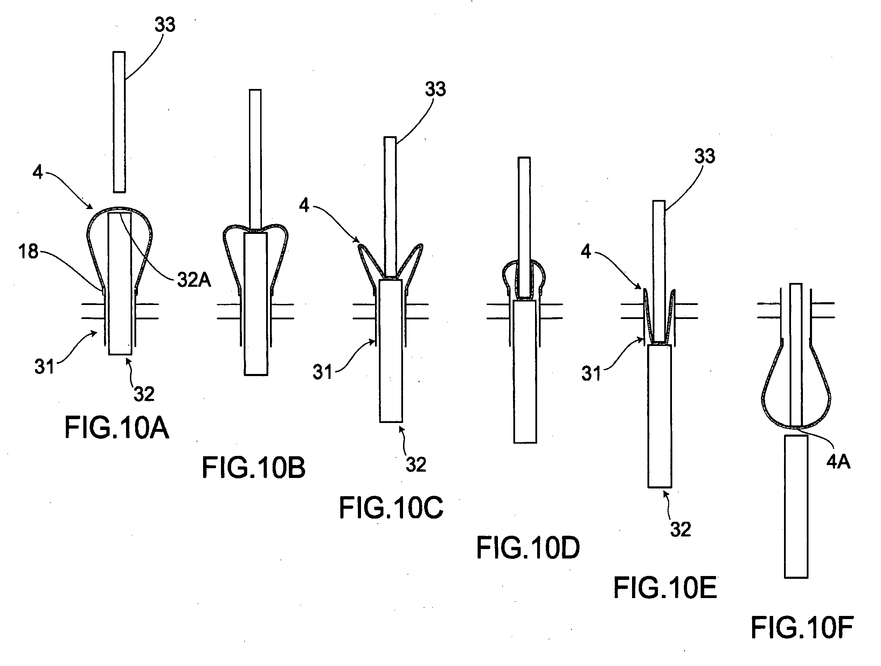

In the present embodiment, an apparatus described hereunder is used to turn the pouch-shaped sheet 4 inside out (e.g. FIGs.10).

This apparatus includes: a cylindrical portion 31 allowing the pouch-shaped sheet 4 (expected to be turned inside out) to pass therethrough; a pole-shaped supporting member 32 supporting the pouch-shaped sheet 4 from underneath; and a push-out unit serving to push out the pouch-shaped sheet 4 downward.

Although the aforementioned push-out unit may, for example, be a blowing device capable of pushing out the pouch-shaped sheet 4 downward via air blow, the present embodiment employs a rod-shaped push-out rod 33 as the push-out unit.

In the beginning, the pouch-shaped sheet 4 with the rough surface 6 formed on the inner side thereof is to be placed on one end (upper end) of the cylindrical portion 31, by allowing the opening section 18 of the pouch-shaped sheet 4 to be pulled over the one end of the cylindrical portion 31. Here, at the one end of the cylindrical portion 31, it is required that the opening section 18 of the pouch-shaped sheet 4 be fitted and moderately supported from within such that the opening section 18 can be smoothly disengaged therefrom when subjected to a proper force.

Next, the pole-shaped supporting member 32 is raised until it has come into contact with the inner side of a front end 4A of the pouch-shaped sheet 4 (e.g. FIG.10A)

That is, the pole-shaped supporting member 32 is to be inserted from the other end (lower end) of the cylindrical portion 31, followed by allowing the front end of the pole-shaped supporting member 32 to protrude outward from the one end (upper end) of the cylindrical portion 31 before coming into contact with the inner side of the front end 4A of the pouch-shaped sheet 4. Particularly, in the present embodiment, a front end 32A of the pole-shaped supporting member 32 is substantially formed into a rectangular shape that has chamfered corners and is extended in the width direction of the pouch-shaped sheet 4.

The aforementioned push-out rod 33 as the push-out unit is then used to lower the pole-shaped supporting member 32 while applying a downward force to the front end 4A of the pouch-shaped sheet 4. In this way, the front end of the pouch-shaped sheet 4 is caused to be folded into the inner side of the pouch-shaped sheet 4.

Furthermore, since the pole-shaped supporting member 32 is lowered while the downward force is being applied to the front end 4A of the pouch-shaped sheet 4, the pouch-shaped sheet 4 is eventually pushed into the cylindrical portion 31, thus allowing the pouch-shaped sheet 4 to be turned inside out (e.g. FIG. 10B to FIG.10E).

In the end, the pouch-shaped sheet 4 thus turned inside out is ejected from the lower end of the cylindrical portion 31, thus completing the step of turning the pouch-shaped sheet 4 inside out (e.g. FIG. 10F).

The reason that the pouch-shaped sheet 4 needs to be turned inside out as a pouch is because it has to be tucked inward such that the burr or the like of the resultant fused portion 17 is not exposed on the outer surface, the resultant fused portion 17 being received inside the pouch-shaped sheet 4. In addition, the resultant fused portion 17 is continuously formed on the entire circumference of the pouch-shaped sheet 4 except the opening section 18.

Next, a method of forming the entire head portion 1 is described.

As a first method, the head portion 1 may be formed by putting the pouch-shaped sheet 4 with the rough surface 6 facing outward over the core portion 8 that has been formed into the shape of the head in advance. Alternatively, the head portion 1 may also be formed by putting the pouch-shaped sheet 4 with the rough surface 6 facing inward over the core portion 8, while gradually turning the corresponding pouch-shaped sheet 4 inside out.

When forming the head portion 1 using the first method, a material such as a rubber or the like may also be employed in order to elasticize the pouch-shaped sheet 4.

As a second method, the head portion 1 may be formed by injecting the core portion 8 into the pouch-shaped sheet 4 through mold injection.

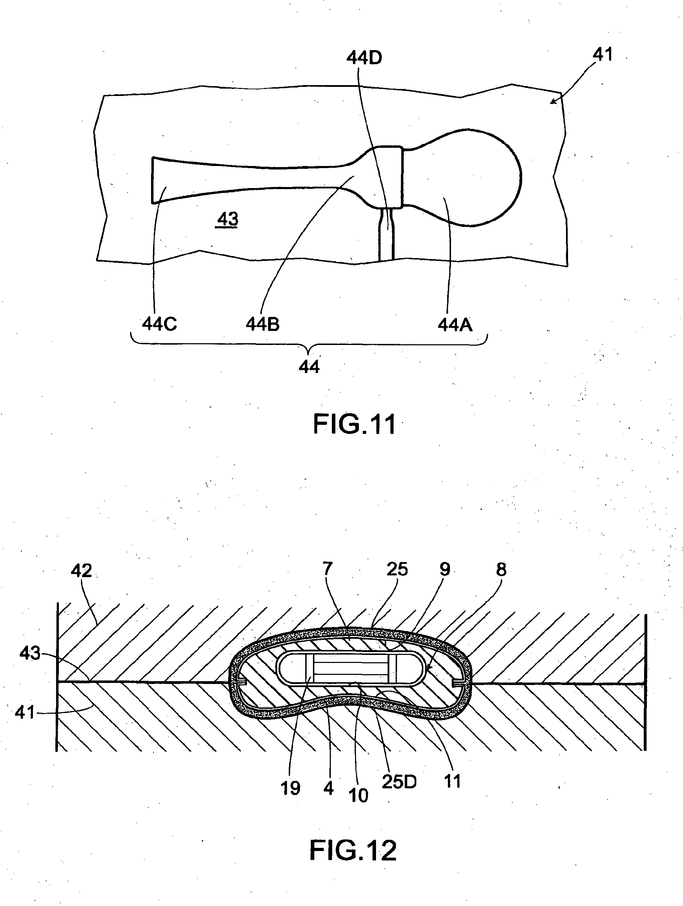

The second method for forming the head portion 1 is described hereunder with reference to FIG.11 to FIG.13.

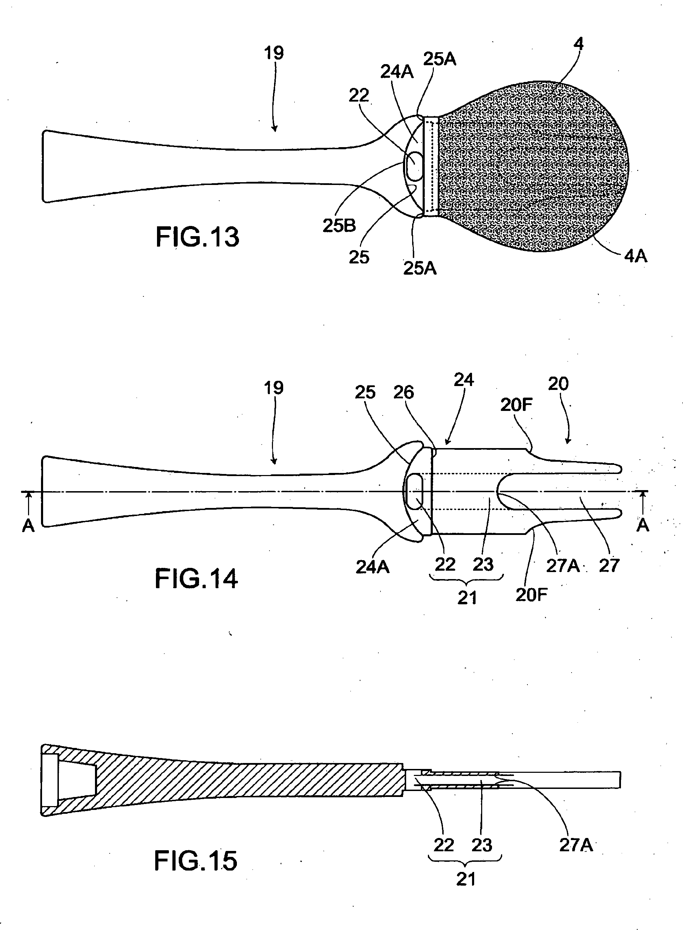

That is, the pouch-shaped sheet 4 is to be pulled over a core base member 19 serving as the core of both the core portion 8 and the head base portion 3 (e.g. FIG.13). The core base member 19 is made of the same synthetic resin of which the rod-shaped grip 2 is made, and the pouch-shaped sheet 4 is actually put over an attachment portion 24 provided on the front end section of the core base member 19.

In the present embodiment, the attachment portion 24 of the core base member 19 includes: a biforked protrusion 20 protruding forward and capable of abutting against the inner side of the front end 4A of the pouch-shaped sheet 4; and a inflow hole 21 serving as a passage for flowing a molten resin Y into the front end section of the attachment portion 24.

In the present embodiment, the core base member 19 is integrally formed together with the head base portion 3 and a part of the rod-shaped grip 2 (e.g. FIG.11, FIG.12).

The protrusion 20 servers to: prevent the pouch-shaped sheet 4 from being placed into molds 41, 42 in a bended or folded fashion; and eventually prevent the molten resin Y from breaking through the pouch-shaped sheet 4 and then leaking outward as the pouch-shaped sheet 4 fails to fully expand within the molds 41, 42 when pouring the molten resin Y.

The inflow hole 21 includes: a through hole 22 passing through the base section of the core base member 19 from the front side to the back side thereof; and a communication through hole 23 connecting the through hole 22 to the front end section of the core base member 19.

In addition, as shown in FIG.16, a first step portion 25 is circumferentially provided on the core base member 19 in a manner such that the front end section of this first step portion 25 is formed thin in the vertical direction, and the width thereof is formed narrow in the left-right direction. Further, the first step portion 25, when viewed from top, is formed into a curved shape with a left and right regions 25A, 25A thereof being located closer to the front end than a central region 25B in the left-right direction. Furthermore, an attachment base-end region 24A of the attachment portion 24 is provided on the front end section of the first step portion 25. The attachment base-end region 24A is formed into the shape of a plate having a substantially constant thickness and width. A second step portion 26 is provided on the front end of the attachment base-end region 24A, and the aforementioned protrusion 20 is so formed that it protrudes from the second step portion 26 toward the front end. Particularly, the protrusion 20 is formed thinner and narrower than the attachment base-end region 24A due to the second step portion 26.

Provided on the front end of the protrusion 20 are a left and right front end portions 20A, 20A composing a biforked shape as a whole. A clearance 27 is provided between these front end protrusions 20A, 20A. Moreover, the front end of the communication through hole 23 is communicated with a bottom portion 27A located on the base end section of the clearance 27.

Here, provided on a left and right outer edges of the protrusion 20 are guiding edge regions 20F, 20F whose front end sections are formed narrower. Due to these left and right guiding edge regions 20F, 20F, the outer edges of the left and right front end protrusions 20A, 20A are formed closer to each other and thereby have a distance therebetween that is smaller than the width of the base end of the protrusion 20. This allows the left and right front end protrusions 20A, 20A to be easily inserted into the opening section 18 of the pouch-shaped sheet 4.

Further, the outer perimeter of the attachment base-end region 24A is substantially identical to the inner perimeter of the opening section 18. Therefore, after inserting the front end protrusions 20A, 20A into the opening section 18, the left and right guiding edge regions 20F, 20F shall allow the attachment base-end region 24A to be smoothly inserted into the pouch-shaped sheet 4, thereby causing this pouch-shaped sheet 4 to then be positioned as the edge of the opening section 18 abuts against the left and right sections of the first step portion 25. As shown in FIG.16, the opening section 18 is to be attached around the attachment base-end region 24A, specifically at the location where the pouch-shaped sheet 4 is positioned as above.

After attaching the pouch-shaped sheet 4 to the attachment portion 24, the core portion 8 and the head base portion 3 are to be molded using the aforementioned molds 41, 42 that are shown in FIG.11, FIG.12. Molding performed using the molds 41, 42 is as follows. That is, the core portion 8 and the head base portion 3 are integrally molded with the pouch-shaped sheet 4 and the core base member 19 being placed in the molds.

A cavity 44 as a molding space formed by the halved molds 41, 42 along the division surface 43, includes: a first section 44A for molding the core portion 8 and eventually the head portion 1; a second section 44B for molding the head base portion 3; and a third section 44C for holding a part of the rod-shaped grip 2. Further, formed on the side region of the second section 44B is a feed opening section 44D for feeding the molten resin Y. Here, when the molds are closed, the feed opening section 44D is located slightly ahead of the through hole 22 toward the front end.

In such case, as shown in FIG.17, once the molds 41, 42 are closed, formed in the second section 44B is a molding cavity 45 for molding the sealing portion 3A between the attachment base-end region 24A and the second section 44B.

In the first section 44A, the molten resin Y flows into the pouch-shaped sheet 4 from the feed opening section 44D through the inflow hole 21, thereby expanding the pouch-shaped sheet 4, thus making it possible to mold the core portion 8 and eventually the head portion 1.

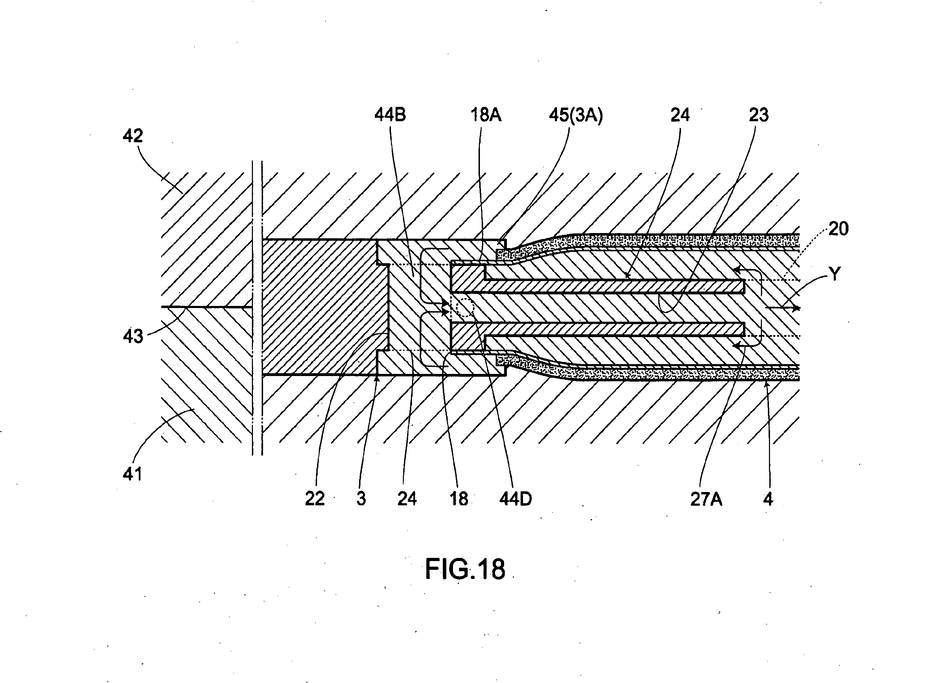

In particular, as shown in FIG.17 and FIG.18, when feeding the molten resin Y into the cavity 44 from the feed opening section 44D to perform mold injection, the molten resin Y shall pass through the second section 44B having the molding cavity 45 before flowing successively into the through hole 22 and then the communication through hole 23, thereby filling the interior of the pouch-shaped sheet 4 from the front end of the communication through hole 23, thus allowing the pouch-shaped sheet 4 to be formed into the shape of the first section 44A of the cavity 44. Subsequently, as the molten resin Y hardens, there is formed the core portion 8 integrally made of both the attachment portion 24 and the resin in the first section 44A.

Here, since the molten resin Y slightly enters the fibers of the pouch-shaped sheet 4 when hardening, the core portion 8 is allowed to tightly adhere to the pouch-shaped sheet 4.

In the second section 44B, the molten resin Y supplied from the feed opening section 44D takes in the end portion of the opening section 18 of the pouch-shaped sheet 4, thus molding the head base portion 3.

Here, since the end portion of the opening section 18 is taken in, water can be prevented from infiltrating through the end portion of the opening section 18.

Moreover, the core base member 19 is tightly received in the third section 44C such that the molten resin Y does not flow thereinto.

If forming the head portion 1 using the second method, there is no need to consider the ease of putting the pouch-shaped sheet 4 over the core portion 8. Therefore, the pouch-shaped sheet 4 can be formed into the shape of a pouch having a front end portion that is even broader than the opening section 18; or a protrusion(s), for example, can be easily formed for the purpose of achieving a certain effect, thus improving the degree of freedom of the shape of the head portion 1.

The maximum width dimension of the pouch-shaped sheet 4 in the left-right direction is 15 to 30 mm, and is about 25 mm in the present embodiment. Meanwhile, the width dimension of the opening section 18 in the left-right direction is 9 to 19 mm, and is about 14 mm in the present embodiment. Particularly, the maximum width dimension of the pouch-shaped sheet 4 in the left-right direction is larger than the width dimension of the opening section 18 in the left-right direction by 5 mm or more. Here, these dimensions are obtained with the upper and lower pieces of the sheet material 5 being laminated on top of each other. However, when the difference between the aforementioned maximum width dimension in the left-right direction and the width dimension of the opening section in the left-right direction is excessively large, it becomes difficult to not only put the pouch-shaped sheet 4 over the core portion 8 if employing the first method, but also turn the pouch-shaped sheet 4 inside out if employing the second method. Furthermore, when the width dimension of the opening section 18 in the left-right direction is large, the head base portion 3 becomes large also. In this sense, it is preferred that the dimensions of the aforementioned range be employed.

Further, the second method allows the core portion 8 to tightly adhere to the inner side of the pouch-shaped sheet 4. Since the core portion 8 tightly adheres to the inner side of the pouch-shaped sheet 4, the inner side of the pouch-shaped sheet 4 is made waterproof, thereby making it possible to inhibit bacteria inside the pouch-shaped sheet 4 from proliferating, thus sanitizing the intraoral cleaner.

Furthermore, since the core portion 8 tightly adheres to the inner side of the pouch-shaped sheet 4, the inner side of the pouch-shaped sheet 4 is made waterproof. Therefore, while sheets 104,105 of a conventional tongue cleaner have their rear surfaces coated with a urethane resin for the purpose of obtaining a waterproof property, the rear surfaces (surfaces opposite to the rough surfaces 6) of the pouch-shaped sheet 4 of the present invention need not be coated in advance. For this reason, the pouch-shaped sheet 4 can be formed soft enough to be easily turned inside out.

Furthermore, the linear members 13 that are vertically, for example, knitted onto the sheet materials 5, may stretch and then fall out when pulled with a force. This can be prevented by performing coating using the urethane resin. However, in the present embodiment, since the molten resin Y hardens inside the pouch-shaped sheet 4, the linear members 13 can, through the rear surfaces of the sheet material 5, adhere and be fixed to the core portion 8 made of the hardened molten resin Y. Therefore, the linear members 13 can be prevented from falling out, thus making it possible to omit the conventional step of performing coating.

Furthermore, as compared to a head portion 101 of the conventional tongue cleaner, the head portion 1 of the intraoral cleaner of the present invention has the whole surface thereof covered by the soft loop-shaped linear members 13. Particularly, since the head portion 1 is not equipped with a ring portion, the head portion 1 can be downsized. Since the head portion 1 has the whole surface thereof covered by the soft linear members, even small children or female users with smaller mouths shall find the cleaner easy to use; hard to induce a nauseous feeing at the time of use; and capable of preventing an unpleasant feeling inflicted as a hard portion(s) come into contact with teeth.

Furthermore, since the head portion 1 of the intraoral cleaner of the present invention is formed thin and small, even those who are incapable of widely opening their mouths may use the cleaner of the present invention. Since the head portion 1 has the whole surface thereof covered by the soft linear members, even those who are having their tongues cleaned by others can be prevented from developing the unpleasant feeling inflicted as a hard portion(s) come into contact with their teeth. Therefore, the intraoral cleaner of the present invention can also be used by caregivers to perform oral care on those in need of nursing care, such oral care including, for example, intraoral massage aimed at inducing saliva.

Furthermore, since the width dimension of the head base portion 3 in the left-right direction is smaller than the maximum width dimension of the head portion 1 in the left-right direction, a space inside the mouth is ensured when using the cleaner. Particularly, since the side surface of the front end portion 1A of the head portion 1 is formed into a curved surface, a cleaning effect superior to that of the conventional tongue cleaner can be achieved by moving the side surface of the front end portion 1A along the tongue or the like. For example, it is now possible to perform partial cleaning using the side surface of the front end portion 1A with the head portion 1 being held vertically against the surface of the tongue. In addition, it is now also possible to perform cleaning using the left and right side surfaces of the head portion 1 which were not suitable for use before.

Furthermore, the plurality of the thin needles 52 as hooking members radially protrude from the cylindrical roller 51. These needles 52 serve to individually hook the linear members 13 so as to apply a tensile force thereto. In this way, the linear members 13 are caused to stretch through plastic deformation. Subsequently, a plastic forming step is performed to remove the tensile force, thus making it possible to easily form the linear members 13 having the concavities and convexities whose orientations and shapes are not identical to one another. Moreover, since the reversing apparatus used to turn the pouch-shaped sheet 4 inside out includes: the cylindrical roller 51; the pole-shaped supporting member 32 as a supporting unit; and a push-out unit, the pouch-shaped sheet 4 whose left-right dimension of its opening section is smaller than its maximum width dimension in the left-right direction can be easily turned inside out. Particularly, since the front end 32A of the pole-shaped supporting member 32 is substantially formed into a rectangular shape extended in the width direction of the pouch-shaped sheet 4, the front end 32A is capable of stably supporting a pouch-shaped object. In addition, since no linear member 13 is provided on the entire circumference of the band-shaped region 18A of the opening section 18, there can achieved a superior an adhesiveness of the sealing portion 3A to the resin. Since provided on the front end of the core base member 19 is the attachment portion 24 to be inserted into the pouch-shaped sheet 4, mold injection can be stably performed with the shape of the pouch-shaped sheet 4 being maintained by the attachment portion 24. The inflow hole 21 though which the molten resin Y passes, includes: the vertical through hole 22; and the communication through hole 23 communicated with the through hole 22 in an orthogonal fashion, thereby allowing the molten resin Y to smoothly flow into the cavity 44 having the molding cavity 45 for molding the sealing portion. Since the attachment base-end region 24A serves to maintain the shape of the pouch-shaped sheet 4 prior to performing molding, there can be obtained the core portion 8 that is integrally formed together with the hardened molten resin Y.

However, the present invention is not limited to the aforementioned embodiment. In fact, various modified embodiments are available within the scope of the present invention. For example, in the present embodiment, the two pieces of the sheet material that are fused to each other are integrally cut out and then separated as a whole in the regions of the resultant fused portion(s) such that the pouch-shaped sheets can be formed. However, the two pieces of the sheet material may actually be separated as a whole outside the resultant fused portion(s). Further, the resin used shall not be limited to that of the aforementioned embodiment. As a matter of fact, various synthetic resins can be used. Here, a heat-curable resin may be used if performing injection molding.

## Claims

1. An intraoral cleaner comprising:
a rod-shaped grip (2); and
a thin and flat head portion (1) provided on a front end of said rod-shaped grip (2), which includes:
a pouch-shaped sheet (4); and
a core portion (8) filling an interior of said pouch-shaped sheet (4), said pouch-shaped sheet (4) having, on an entire outer surface thereof, a plurality of loop-shaped linear members (13) for scraping out dirt inside an oral cavity;
**characterized in that** said pouch-shaped sheet (4) comprising two pieces of sheet material (5) further has a joint section (17) formed by a fused portion of the two pieces of sheet material (5) on an inner side of the pouch-shaped sheet (4).

2. The intraoral cleaner according to claim 1, **characterized in that** said pouch-shaped sheet (4) and said core portion (8) are tightly adhered to each other.

3. The intraoral cleaner according to claim 1 or 2, **characterized in that** said pouch-shaped sheet (4) is formed into the shape of a pouch having a portion broader than an opening section (18).

4. The intraoral cleaner according to claim 2, **characterized in that** said pouch-shaped sheet (4) and said core portion (8) tightly adhere to each other such that said linear members (13) can adhere and be fixed to the inner side of said pouch-shaped sheet (4).

5. The intraoral cleaner according to claim 1, **characterized in that** said pouch-shaped sheet (4) further has a band-shaped region (18A) that is formed in an opening section (18) and is free from said loop-shaped linear members (13), said band-shaped region (18A) being sealed by a sealing portion (3A).

6. The intraoral cleaner according to claim 5, **characterized in that** said head portion (1) and said rod-shaped grip (2) are provided with a head base portion (3) formed therebetween, and said sealing portion (3A) is provided on a front end of said head base portion (3).

7. The intraoral cleaner according to claim 1, **characterized in that** said linear members (13) are provided in the order of 300 deniers.

8. The intraoral cleaner according to claim 1, **characterized in that** said linear members (13) are provided in the order of 2,000 loops per 1 cm square on the outer surface of said pouch-shaped sheet (4).

9. A method for manufacturing an intraoral cleaner comprising a rod-shaped grip (2) and a thin and flat head portion (1) provided on a front end of said rod-shaped grip (2) according to claim 1, **characterized in that** said head portion (1) is formed through:
a step of forming said pouch-shaped sheet (4) by placing sheet materials (5) on top of each other with rough surfaces (6) thereof facing each other, said rough surfaces (6) being first surfaces of said sheet materials (5) and having a plurality of linear members (13), and then fusing said sheet materials (5) such that a resultant joint section (17) conforms to the outer shape of the head portion (1);
a step of turning said pouch-shaped sheet (4) inside out; and
a step of forming said core portion (8) inside said pouch-shaped sheet (4) through injection molding.

10. The method according to claim 9, **characterized in that** a sealing portion (3A) and said core portion (8) are formed together in the step of forming the core portion (8), said sealing portion (3A) sealing a band-shaped region (18A) that is formed in an opening section (18) of said pouch-shaped sheet (4) and is free from said loop-shaped linear members (13).

## Patentansprüche

1. Intraoraler Reiniger, der aufweist:
einen stabförmigen Griff (2); und
ein dünnes und flaches Kopfteil (1), das an einem vorderen Ende des stabförmigen Griffs (2) angeordnet ist und das aufweist:
ein taschenförmiges Tuch (4); und
ein Kernstück (8), welches einen Innenraum des taschenförmigen Tuchs (4) ausfüllt, wobei das taschenförmige Tuch (4) auf seiner gesamten äußeren Oberfläche eine Vielzahl schlaufenförmiger Bandelemente (13) aufweist, um Verunreinigungen innerhalb einer Mundhöhle heraus zu schaben;
**dadurch gekennzeichnet, dass** das taschenförmige Tuch (4) welches zwei Teile aus bahnförmigem Material (5) aufweist, weiterhin einen Verbindungsbereich (17) aufweist, der von einem verschweißten Anteil der beiden Teile aus bahnförmigem Material (5) an einer Innenseite des taschenförmigen Tuchs (4) gebildet ist.

2. Intraoraler Reiniger nach Anspruch 1, **dadurch gekennzeichnet, dass** das taschenförmige Tuch (4) und das Kernstück (8) fest aneinander haften.

3. Intraoraler Reiniger nach Anspruch 1 oder 2, **dadurch gekennzeichnet, dass** das taschenförmige Tuch (4) in der Form einer Tasche ausgebildet ist, welche einen Anteil aufweist, der breiter als ein Öffnungsbereich (18) ist.

4. Intraoraler Reiniger nach Anspruch 2, **dadurch gekennzeichnet, dass** das taschenförmige Tuch (4) und das Kernstück (8) fest aneinander haften, so dass die schlaufenförmigen Bandelemente (13) an der Innenseite des taschenförmigen Tuches (4) anhaften und dort befestigt sind.

5. Intraoraler Reiniger nach Anspruch 1, **dadurch gekennzeichnet, dass** das taschenförmige Tuch (4) weiterhin einen streifenförmigen Bereich (18A) aufweist, der in einem Öffnungsbereich (18) ausgebildet und frei von den schlaufenförmigen Bandelementen (13) ist, wobei der streifenförmige Bereich (18A) über einen Versiegelungsabschnitt (3A) versiegelt ist.

6. Intraoraler Reiniger nach Anspruch 5, **dadurch gekennzeichnet, dass** das Kopfteil (1) und der stabförmige Griff (2) mit einem dazwischen ausgebildeten Kopfunterteil (3) versehen sind, und wobei der Versiegelungsabschnitt (3A) an einem vorderen Ende des Kopfunterteils (3) bereitgestellt ist.

7. Intraoraler Reiniger nach Anspruch 1, **dadurch gekennzeichnet, dass** die schlaufenförmigen Bandelemente (13) mit im Größenbereich von 300 Denier bereitgestellt sind.

8. Intraoraler Reiniger nach Anspruch 1, **dadurch gekennzeichnet, dass** die schlaufenförmigen Bandelemente (13) im Größenbereich von 2000 Schlaufen pro Quadratzentimeter auf der äußeren Oberfläche des taschenförmigen Tuchs (4) bereitgestellt sind.

9. Verfahren für die Herstellung eines intraoralen Reinigers, der einen stabförmigen Griff (2) und ein dünnes und flaches Kopfteil (1) aufweist, das auf einem vorderen Ende des stabförmigen Griffs (2) gemäß Anspruch 1 bereitgestellt ist, **dadurch gekennzeichnet, dass** der Kopfteil (1) wie folgt ausgebildet wird:
ein Schritt, bei dem das taschenförmige Tuch (4) durch Anordnen der bahnförmigen Materialien (5) übereinander und mit den rauhen Oberflächen (6) dieser einander zugewandt ausgebildet wird, wobei die rauhen Oberflächen (6) erste Oberflächen der bahnförmigen Materialien (5) sind, und wobei sie eine Vielzahl schlaufenförmiger Bandelemente (13) aufweisen, und wobei die bahnförmigen Materialien (5) dann verschweißt werden, so dass sich ein resultierender Verbindungsbereich (17) ergibt, der der äußeren Form des Kopfteils (1) entspricht;
ein Schritt, bei dem die Innenseite des taschenförmigen Tuchs (4) nach außen gestülpt wird; und
ein Schritt, bei dem das Kernstück (8) innerhalb des taschenförmigen Tuchs (4) mit Hilfe von Spritzgießen ausgebildet wird.

10. Verfahren nach Anspruch 9, **dadurch gekennzeichnet, dass** ein Versiegelungsabschnitt (3A) und das Kernstück (8) gemeinsam während des Ausbildungsschritts des Kernstücks (8) ausgebildet werden, wobei der Versiegelungsabschnitt (3A) einen streifenförmigen Bereich (18A) versiegelt, der in einem Öffnungsbereich (18) des taschenförmigen Tuchs (4) ausgebildet wird und der frei von den schlaufenförmigen Bandelementen (13) ist.

## Revendications

1. Dispositif de nettoyage intra-buccal comprenant :
une poignée en forme de tige (2) ; et
une partie de tête mince et plate (1) prévue sur une extrémité avant de ladite poignée en forme de tige (2), qui comprend :
une feuille en forme de sachet (4) ; et
une partie de noyau (8) remplissant un intérieur de ladite feuille en forme de sachet (4), ladite feuille en forme de sachet (4) comportant, sur une surface extérieure entière de celle-ci, une pluralité d'éléments linéaires en forme de boucles (13) pour racler la saleté à l'intérieur d'une cavité buccale ;
**caractérisé en ce que** ladite feuille en forme de sachet (4) comprenant deux pièces de matériau en feuille (5) comporte en outre une section de jonction (17) formée par une partie fusionnée des deux pièces de matériau en feuille (5) d'un côté intérieur de la feuille en forme de sachet (4).

2. Dispositif de nettoyage intra-buccal selon la revendication 1, **caractérisé en ce que** ladite feuille en forme de sachet (4) et ladite partie de noyau (8) adhèrent étroitement l'une à l'autre.

3. Dispositif de nettoyage intra buccal selon la revendication 1 ou 2, **caractérisé en ce que** ladite feuille en forme de sachet (4) est réalisée en la forme d'un sachet comportant une partie plus large qu'une section d'ouverture (18).

4. Dispositif de nettoyage intra-buccal selon la revendication 2, **caractérisé en ce que** ladite feuille en forme de sachet (4) et ladite partie de noyau (8) adhèrent étroitement l'une à l'autre de sorte que lesdits éléments linéaires (13) peuvent adhérer et être fixés au côté intérieur de ladite feuille en forme de sachet (4).

5. Dispositif de nettoyage intra-buccal selon la revendication 1, **caractérisé en ce que** ladite feuille en forme de sachet (4) comporte en outre une région en forme de bande (18A) qui est formée dans une section d'ouverture (18) et qui est exempte desdits éléments linéaires en forme de boucles (13), ladite région en forme de bande (18A) étant fermée hermétiquement par une partie de fermeture étanche (3A).

6. Dispositif de nettoyage intra-buccal selon la revendication 5, **caractérisé en ce que** ladite partie de tête (1) et ladite poignée en forme de tige (2) sont pourvues d'une partie de base de tête (3) formée entre elles, et ladite partie de fermeture étanche (3A) est prévue sur une extrémité avant de ladite partie de base de tête (3).

7. Dispositif de nettoyage intra-buccal selon la revendication 1, **caractérisé en ce que** lesdits éléments linéaires (13) sont prévus dans la plage de 300 deniers.

8. Dispositif de nettoyage intra-buccal selon la revendication 1, **caractérisé en ce que** lesdits éléments linéaires (13) sont prévus dans la plage de 2.000 boucles par cm² sur la surface extérieure de ladite feuille en forme de sachet (4).

9. Procédé pour la fabrication d'un dispositif de nettoyage intra-buccal comprenant une poignée en forme de tige (2) et une partie de tête mince et plate (1) prévue sur une extrémité avant de ladite poignée en forme de tige (2) selon la revendication 1, **caractérisé en ce que** ladite partie de tête (1) est formée par :
une étape de formation de ladite feuille en forme de sachet (4) en plaçant des matériaux en feuille (5) l'un au-dessus de l'autre, les surfaces rugueuses (6) de ceux-ci se faisant mutuellement face, lesdites surfaces rugueuses (6) étant des premières surfaces desdits matériaux en feuille (5) et comportant une pluralité d'éléments linéaires (13), et de fusion ensuite desdits matériaux en feuille (5) de sorte qu'une section de jonction (17) résultante soit conforme à la forme extérieure de la partie de tête (1) ;
une étape de retournement de ladite feuille en forme de sachet (4) de l'intérieur à l'extérieur ; et
une étape de formation de ladite partie de noyau (8) à l'intérieur de ladite feuille en forme de sachet (4) par moulage par injection.

10. Procédé selon la revendication 9, **caractérisé en ce qu'**une partie de fermeture étanche (3A) et ladite partie de noyau (8) sont formées, ensemble, à l'étape de formation de la partie de noyau (8), ladite partie de fermeture étanche (3A) fermant hermétiquement une région en forme de bande (18A) qui est formée dans une section d'ouverture (18) de ladite feuille en forme de sachet (4) et qui est exempte desdits éléments linéaires en forme de boucles (13).
